# EUROPEAN PATENT SPECIFICATION

(11) **EP 0 708 087 B1**
(45) Date of publication and mention of the grant of the patent: **28.07.1999**
(21) Application number: 95307217.0
(22) Date of filing: 11.10.1995
(51) Int. Cl.: C07C 303/30, C07C 303/32, C07C 303/22, C07C 309/73, C07C 309/68, C07C 309/86, C07F 9/40

(54) **Method for making a substituted benzene compound**
Verfahren zur Herstellung einer substituierten Benzol-Verbindung
Procédé pour la préparation d'un composé benzénique

(30) Priority: 18.10.1994 US 324655; 07.09.1995 US 524821
(43) Date of publication of application: 24.04.1996
(73) Proprietor: ROHM AND HAAS COMPANY, Philadelphia, Pennsylvania 19106-2399 (US)
(72) Inventor: Spangler, Lori Ann, Churchville, Pennsylvania 18966 (US); Weaver, Damian Gerard, Lansdale, Pennsylvania 19446 (US)
(74) Representative: Tanner, James Percival

(56) References cited:
- US-A- 5 272 128
- JOURNAL OF ORGANIC CHEMISTRY, vol. 51, no. 14, 11 July 1986 WASHINGTON, DC, US, pages 2833-2835, J.N. BONFIGLIO: 'Directed ortho- lithiation of alkyl arenesulphonates'
- JOURNAL OF ORGANIC CHEMISTRY, vol. 45, no. 18, 29 August 1980 WASHINGTON, DC, US, pages 3728-3729, G.D. FIGULY, ET AL.: 'Ortho lithiations of arenesulphonic acids. New methodology for electrophilic aromatic substitutions'

## Description

The present invention is directed to a method for making a substituted benzene compound, and especially to a method for making a 2,6-disubstituted benzenesulfonate or a 2,6-disubstituted benzenesulfonate further substituted in the 3, 4 or 5-position.

Ortho-metalation of alkyl arenesulfonates to provide 2-substituted and 2,4-disubstituted alkyl arenesulfonates has been reported, see "Directed Ortho-Lithiation of Alkyl Arenesulfonates", J. N. Bonfiglio, *J. Org. Chem.* 1986, 51, 2833-2835. However, Bonfiglio is silent with respect to methods for making a 2,6-substituted alkyl arenesulfonate.

According to the present invention there is provided a method for making a substituted benzene compound, which comprises: reacting a 2-substituted benzenesulfonate, or a 2-substituted benzenesulfonate further substituted in the 3, 4 or 5-position, with a lithium compound to form an intermediate compound; and reacting the intermediate compound with an electrophile to form a 2,6-disubstituted benzenesulfonate or a 2,6-disubstituted benzenesulfonate further substituted in the 3, 4 or 5-position.

The 2,6-disubstituted benzenesulfonates or 2,6-disubstituted benzenesulfonates further substituted in the 3, 4 or 5-position made by the method of the present invention are useful as intermediates in a method for making certain compounds known to be useful as herbicides, for example, the phosphosulfonate compounds disclosed in US-A-5,272,128 and the sulfonyl urea compounds disclosed in US-A-4,127,405.

More specifically, the present invention provides a method for making a substituted benzene compound which comprises: reacting a 2-substituted benzenesulfonate, or a 2-substituted benzenesulfonate further substituted in the 3,4 or 5-position, with a lithium compound to form an intermediate compound; and reacting said intermediate compound with an electrophile, selected from the group consisting of alkyl halides, haloalkyl alkyl ethers, aldehydes, ketones, alkyl sulfates, boron esters, alkyl disulfides, phenyl disulfide, deuterium oxide, dimethylformamide, N-formylpiperidine, carbon dioxide, trialkylsilyl chlorides, and sources of positive halogens, to form a 2,6-disubstituted benzenesulfonate or a 2,6-disubstituted benzenesulfonate further substituted in the 3,4 or 5-position, said 2-substituted benzenesulfonate or said 2-substituted benzenesulfonate further substituted in the 3,4 or 5-position is a compound having the structural formula (1): 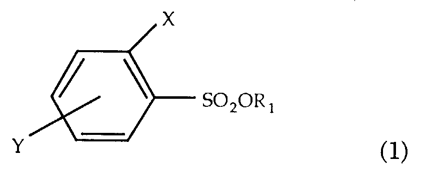 wherein:
R₁ is alkyl, cycloalkyl or phenyl;
X is any group, selected from the group consisting of fluoro, chloro, alkoxy, haloalkyl, haloalkoxy, alkyl, alkylthio, haloalkylthio and N,N-dialkylcarboxamide, that is not reactive with the lithium compound under the reaction conditions used; and
Y is any group, selected from the group consisting of a hydrogen atom, fluoro, chloro, alkoxy, haloalkyl, haloalkoxy, alkylthio and haloalkylthio, in the 3, 4 or 5-position that is not reactive with the lithium compound under the reaction conditions used.

In a preferred embodiment, R₁ is (C₂-C₅)alkyl and, more preferably, isopropyl.

In a preferred embodiment, X is fluoro, chloro, alkoxy, more preferably (C₁-C₃)alkoxy, haloalkyl, more preferably halo(C₁-C₃)alkyl, haloalkoxy, more preferably halo(C₁-C₃)alkoxy, alkyl, more preferably (C₁-C₄)alkyl, alkylthio, more preferably (C₁-C₄)alkylthio, haloalkylthio, more preferably halo(C₁-C₄)alkylthio, or N,N-dialkylcarboxamide, more preferably N,N-di(C₂-C₄)alkylcarboxamide.

In a highly preferred embodiment, X is chloro, fluoro, methoxy, trifluoromethyl, pentafluoroethyl, methylthio, ethylthio or trifluoromethoxy.

In a preferred embodiment, Y is a hydrogen atom, fluoro, chloro, alkoxy, more preferably (C₁-C₃)alkoxy, haloalkyl, more preferably halo(C₁-C₃)alkyl, haloalkoxy, more preferably halo(C₁-C₃)alkoxy, alkylthio, more preferably (C₁-C₄)alkylthio, or haloalkylthio, more preferably halo(C₁-C₄)alkylthio.

In a highly prefered embodiment, Y is a hydrogen atom, chloro, fluoro, methoxy, trifluoromethyl, trifluoromethoxy, pentafluoroethyl, methylthio, ethylthio or trifluoromethylthio.

"Alkyl" means a straight or branched alkyl chain and includes, for example, methyl, ethyl, *n*-propyl, isopropyl, *n*-butyl, *sec*-butyl *tert*-butyl, *n*-pentyl, *n*-hexyl. "Cycloalkyl" means a monocyclic non-aromatic alkyl ring and includes, for example, cyclobutyl, cyclopentyl, cyclohexyi. "Alkoxy" means a linear or branched alkoxy group and includes, for example, methoxy, ethoxy, isopropoxy and *n*-propoxy. "Alkylthio" means a linear or branched alkyl group attached to a sulfur atom and includes, for example, methylthio, ethylthio, isopropylthio and *n*-propylthio. "Haloalkyl" means a linear or branched alkyl group substituted with one or more halogen atoms and includes, for example, trifluoromethyl, perfluoroethyl and 2,2,2-trifluoroethyl. "Haloalkoxy" means a linear or branched alkoxy group substituted with one or more halogen atoms and includes, for example, trifluoromethoxy, perfluoroethoxy and chloromethoxy. "Haloalkylthio" means a linear or branched alkyl group, substituted with one or more halogen atoms, attached to a sulfur atom and includes, for example, trifluoromethylthio, perfluoroethylthio and chloromethylthio. "N,N-Dialkyl carboxamide" means a carboxamide group wherein the nitrogen atom is substituted with two alkyl groups and includes, for example, diethylcarboxamide and diisopropylcarboxamide.

Suitable lithium compounds are those lithium compounds that are sufficiently strong bases to remove a proton having a pKa of 30-37 and include, for example, elemental lithium as well as organolithium compounds such as, for example, (C₁-C₆)alkyl lithiums and aryl lithiums. "(C₁-C₆)alkyl lithium" means an alkyl lithium compound having from 1 to 6 carbon atoms per molecule, and includes, for example, butyl lithium, hexyl lithium and methyl lithium. "Aryl lithium" means an aromatic organolithium species, and includes, for example, phenyl lithium, and thienyl lithium.

While not wishing to be bound by theory, it is believed that the intermediate formed in the first step of the method of the present invention, when the benzenesulfonate reactant used has the above formula (1), is an organolithium intermediate having the structural formula (2): 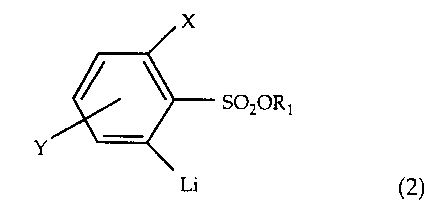 wherein R₁, X and Y are defined as above.

Suitable electrophiles are those compounds that react to form a covalent bond with an anionic intermediate such as compound (2) and that do not contain an acidic proton which can be deprotonated by an anionic intermediate such as compound (2). In a preferred embodiment, the electrophile is selected from the group consisting of alkyl halides, haloalkyl alkyl ethers, aldehydes, ketones, alkyl sulfates, boron esters, alkyl disulfides, phenyl disulfide, deuterium oxide, dimethylformamide, N-formylpiperidine, carbon dioxide, trialkylsilyl chlorides, and sources of positive halogens. Suitable alkyl halides include, for example, iodomethane, iodoethane and iodopropane. Suitable haloalkyl alkyl ethers include, for example, bromomethyl methyl ether. Suitable aldehydes include, for example, formaldehyde, and benzaldehyde. Suitable ketones include, for example, benzophenone. Suitable alkyl sulfates include, for example, dimethylsulfate. Suitable boron esters include, for example, trimethyl borate and triisopropyl borate. Suitable alkyl disulfides include, for example, methyl disulfide, and ethyl disulfide. Suitable trialkylsilyl chlorides include, for example, trimethylsilyl chloride. Suitable sources of positive halogens include, for example, N-fluorobenzenesulfonimide, N-fluoro-O-benzenedisulfonimide, N-fluoropyridinium salts, N-chlorosuccinimide, and 2,2,2-trifluoroethyl iodide.

Any anhydrous, aprotic solvent may be used in the steps wherein the 2-substituted benzenesulfonate or the 2-substituted benzenesulfonate further substituted in the 3, 4 or 5-position is reacted with a lithium compound to form an intermediate and the intermediate is reacted with an electrophile to form the 2,6-disubstituted benzenesulfonate or the 2,6-disubstituted benzenesulfonate further substituted in the 3, 4 or 5-position. Suitable aprotic solvents include, for example, ethers such as, for example, diethyl ether and ethylene glycol dimethyl ether, including cyclic ethers such as, for example, tetrahydrofuran and dioxane, and alkanes, such as, for example, hexane, heptane and pentane, and aromatic solvents such as, for example, cumene, as well as mixtures thereof. In a preferred embodiment, the solvent is tetrahydrofuran or a mixture of tetrahydrofuran and hexane. In a more highly preferred embodiment, the solvent is a mixture of tetrahydrofuran and hexane that includes from 1 to 4 milliliters (mL) of tetrahydrofuran per millimole (mmole) of the 2-substituted benzenesulfonate or the 2-substituted benzenesulfonate further substituted in the 3, 4 or 5-position and from 0.25 to 3 mL of hexane per mmole of the 2-substituted benzenesulfonate or the 2-substituted benzenesulfonate further substituted in the 3, 4 or 5-position.

In a preferred embodiment, an oxygen-free atmosphere is used in the steps wherein the 2-substituted benzenesulfonate or the 2-substituted benzenesulfonate further substituted in the 3, 4 or 5-position is reacted with a lithium compound to form an intermediate and the intermediate is reacted with an electrophile to form the 2,6-disubstituted benzenesulfonate or the 2,6-disubstituted benzenesulfonate further substituted in the 3, 4 or 5-position.

The step of the present method wherein the 2-substituted benzenesulfonate or the 2-substituted benzenesulfonate further substituted in the 3, 4 or 5-position is reacted with a lithium compound to form an intermediate is conducted at any convenient temperature and is preferably conducted at a temperature of from about -78°C to about 0°C.

The step of the present method wherein the intermediate is reacted with an electrophile to form the 2,6-disubstituted benzenesulfonate or the 2,6-disubstituted benzenesulfonate further substituted in the 3, 4 or 5-position is conducted at any convenient temperature at which the intermediate is stable and is preferably conducted at a temperature of from about -78°C to room temperature, i.e., about 25°C.

Preferably, the 2,6-disubstituted benzenesulfonate or the 2,6-disubstituted benzenesulfonate further substituted in the 3, 4 or 5-position, made by the method of the present invention, is a compound having the structural formula (3): 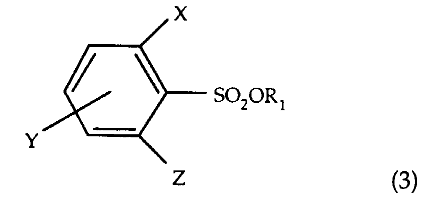 wherein R₁, X and Y are defined as above and Z is the residue of the electrophile.

In a preferred embodiment, Z is alkyl, more preferably (C₁-C₆)alkyl, alkoxy alkyl, methyleneoxy, substituted methyleneoxy, boronic acid, alkylthio, more preferably (C₁-C₃)alkylthio, phenyl, a deuterium atom, formyl, carboxyl, trialkylsilyl, more preferably tri(C₁-C₄)alkylsilyl, or halo.

It is noteworthy that the method of the present invention allows selective preparation of a 2,6-disubstituted benzenesulfonate or a 2,6-disubstituted benzenesulfonate further substituted in the 3, 4 or 5-position from a 2-substituted benzenesulfonate, or a 2-substituted benzenesulfonate further substituted in the 3, 4 or 5-position, wherein the group in the 2 position can be a strongly ortho-directing group, such as, for example, trifluoromethyl, methoxy, trifluoromethoxy, and fluoro.

The 2,6-disubstituted benzenesulfonate or the 2,6-disubstituted benzenesulfonate further substituted in the 3, 4 or 5-position product may be isolated from the reaction mixture by known techniques, such as, for example, extraction.

The steps of the present invention wherein the 2-substituted benzenesulfonate or the 2-substituted benzenesulfonate further substituted in the 3, 4 or 5-position is reacted with a lithium compound to form an intermediate and the intermediate is reacted with an electrophile to form the 2,6-disubstituted benzenesulfonate or the 2,6-disubstituted benzenesulfonate further substituted in the 3, 4 or 5-position typically produces a mixture of the desired 2,6-disubstituted benzenesulfonate or the 2,6-disubstituted benzenesulfonate further substituted in the 3, 4 or 5-position, and a small amount of the unreacted 2-substituted benzenesulfonate or the 2-substituted benzenesulfonate further substituted in the 3, 4 or 5-position starting material. In a preferred embodiment, the product mixture is isolated from the unreacted material by: selectively hydrolyzing the 2,6-disubstituted benzenesulfonate or the 2,6-disubstituted benzenesulfonate further substituted in the 3, 4 or 5-position of the product mixture to form a salt of the 2,6-disubstituted benzenesulfonate or a salt of the 2,6-disubstituted benzenesulfonate further substituted in the 3, 4 or 5-position; partitioning the product mixture between an aqueous phase and an organic phase; and isolating the 2,6-disubstituted benzenesulfonate or the 2,6-disubstituted benzenesulfonate further substituted in the 3, 4 or 5-position salt from the aqueous phase.

In a preferred embodiment, the 2,6-disubstituted benzenesulfonate or the 2,6-disubstituted benzenesulfonate further substituted in the 3, 4 or 5-position is selectively hydrolyzed by adding a source of hydroxyl anions, such as, for example, an aqueous solution of a metal or ammonium hydroxide or a metal carbonate, to the mixture of the 2,6-disubstituted benzenesulfonate or the 2,6-disubstituted benzenesulfonate further substituted in the 3, 4 or 5-position and the 2-substituted benzenesulfonate or the 2-substituted benzenesulfonate further substituted in the 3, 4 or 5-position. In a more highly preferred embodiment, the 2,6-disubstituted benzenesulfonate or the 2,6-disubstituted benzenesulfonate further substituted in the 3, 4 or 5-position is selectively hydrolyzed by adding from about one equivalent to about three equivalents of hydroxide per mole of the 2,6-disubstituted benzenesulfonate or the 2,6-disubstituted benzenesulfonate further substituted in the 3, 4 or 5-position to the mixture of the 2,6-disubstituted benzenesulfonate or the 2,6-disubstituted benzenesulfonate further substituted in the 3, 4 or 5-position and the 2-substituted benzenesulfonate or the 2-substituted benzenesulfonate further substituted in the 3, 4 or 5-position.

The 2,6-disubstituted benzenesulfonate or the 2,6-disubstituted benzenesulfonate further substituted in the 3, 4 or 5-position may be further reacted according to known techniques, for example, the 2,6-disubstituted benzenesulfonate or the 2,6-disubstituted benzenesulfonate further substituted in the 3, 4 or 5-position may be:
desulfonated, for example, by treatment with sulfuric acid and a catalytic metal salt, such as a mercury salt, to form the corresponding 1,3-disubstituted benzene or the corresponding 1,3-disubstituted benzene further substituted in the 4, 5 or 6- position;
hydrolyzed, for example, by treatment with aqueous acid or aqueous base followed by acid, to form the corresponding 2,6-disubstituted benzenesulfonic acid or a salt of a 2,6-disubstituted benzenesulfonic acid, or the corresponding 2,6-disubstituted benzenesulfonic acid further substituted in the 3,4 or 5-position or a salt of a 2,6-disubstituted benzenesulfonic acid further substituted in the 3, 4 or 5-position; or
converted, for example, by treatment with phosphorus oxychloride, to the corresponding 2,6-disubstituted benzenesulfonyl chloride or corresponding 2,6-disubstituted benzenesulfonyl chloride further substituted in the 3, 4 or 5-position.

In a preferred embodiment, the 2,6-disubstituted benzenesulfonate or the 2,6-disubstituted benzenesulfonate further substituted in the 3, 4 or 5-position is made according to the above described method, the 2,6-disubstituted benzenesulfonate or the 2,6-disubstituted benzenesulfonate further substituted in the 3, 4 or 5-position so formed is hydrolyzed and isolated as the corresponding 2,6-disubstituted benzenesulfonate or the 2,6-disubstituted benzenesulfonate further substituted in the 3, 4 or 5-position salt, the salt is then converted to the corresponding 2,6-disubstituted benzenesulfonyl chloride or corresponding 2,6-disubstituted benzenesulfonyl chloride further substituted in the 3, 4 or 5-position and the 2,6-disubstituted benzenesulfonyl chloride or corresponding 2,6-disubstituted benzenesulfonyl chloride further substituted in the 3, 4 or 5-position is then reacted with a hydroxymethylphosphorus compound to form a phosphosulfonate compound.

In a highly preferred embodiment of the present invention, the 2,6-disubstituted benzenesulfonyl chloride or corresponding 2,6-disubstituted benzenesulfonyl chloride further substituted in the 3, 4 or 5-position is reacted with a hydroxymethylphosphorus compound of structural formula (4): 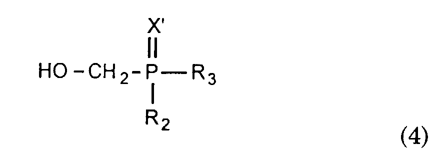 wherein:
X' is an oxygen or a sulfur atom, and
R₂ and R₃ are each independently alkyl, alkoxy, alkylthio, alkenyloxy, alkynyloxy, haloalkoxy, cyanoalkoxy, alkoxyalkoxy, cycloalkyloxy, cycloalkylalkoxy, alkylideneiminooxy, chloro, amino, phenyl or phenoxy groups, wherein the alkyl, alkoxy, alkylthio, alkenyloxy, alkynyloxy, cycloalkyloxy, cycloalkylalkoxy, alkylideneiminooxy, amino, phenyl and phenoxy groups are unsubstituted or substituted; or when R₂ and R₃ are both alkoxy, R₂ and R₃ may, taken together with the phosphorus atom, form a six-membered oxygen-containing ring, to form a phosphosulfonate compound of the structural formula (5): 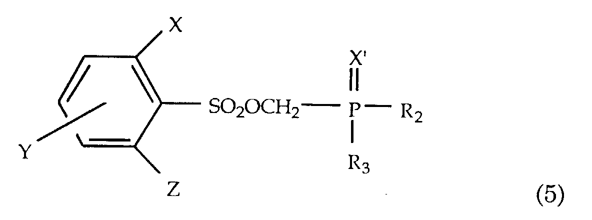 wherein X, X', Y, Z, R₂ and R₃ are each defined as set forth above. In this embodiment Z is preferably alkyl, methyleneoxy, substituted methyleneoxy, boronic acid, alkylthio, a deuterium atom, formyl, carboxyl, trialkylsilyl or halo.

"Alkenyloxy" means an oxygen atom substituted with a straight or branched alkenyl chain and includes, for example, allyloxy. "Alkynyloxy" means an oxygen atom substituted with a straight or branched alkynyl chain and includes, for example, propargyloxy. "Cyanoalkoxy" means a straight or branched alkoxy chain substituted with a cyano group and includes, for example, cyanomethoxy. "Cycloalkyloxy" means an oxygen atom substituted with a cycloalkyl group and includes, for example, cyclobutoxy and cyclopentoxy. "Cycloalkylalkoxy" means a straight or branched alkoxy chain substituted with a cycloalkyl group and includes, for example, cyclopropylmethoxy. "Alkylideneiminooxy" refers an alkyl group doubly bonded to a nitrogen atom which is in turn bonded to an oxygen atom and includes, for example, isopropylideneiminooxy.

Reaction between the 2,6-disubstituted benzenesulfonyl chloride or corresponding 2,6-disubstituted benzenesulfonyl chloride further substituted in the 3, 4 or 5-position and the hydroxymethylphosphorus compound can be carried out under various conditions, for example, in the presence of an organic solvent and an amine base; or in a phase transfer catalyzed reaction wherein the coupling takes place in a two-phase solvent system in the presence of an aqueous base solution and a phase transfer catalyst such as triethylbenzylammonium chloride; or the anion of the hydroxymethylphosphorus compound can be formed with a strong base such as sodium hydride or sodium, then the anion reacted with the 2,6-disubstituted benzenesulfonyl chloride or corresponding 2,6-disubstituted benzenesulfonyl chloride further substituted in the 3, 4 or 5-position, as disclosed in US-A-5,272,128.

A first phosphosulfonate compound of structural formula (5) wherein R₂ is an alkoxy group and R₃ is an alkoxy or an alkyl group can be converted to a second phosphosulfonate of structural formula (5) that is substituted with, for example, an amino, alkylthio or an alkoxy group, the alkoxy group being different from that of the first phosphosulfonate compound, via a phosphonyl chloride or phosphinoyl chloride intermediate by, for example, treating the first phosphosulfonate compound with, for example, phosphorus pentachloride or thionyl chloride, to form the phosphonyl chloride or phosphinoyl chloride intermediate and then treating the intermediate with, for example, an alcohol, a disubstituted amine or an alkyl mercaptan to yield the second phosphonyl chloride compound.

The following Examples and Tables are provided merely to illustrate some embodiments of the method of the present invention and the compounds resulting therefrom and are not intended to limit the scope of the present invention.

### Example 1A: Preparation of isopropyl 2-trifluoromethyl-6-methylbenzenesulfonate

A solution of 400 grams (g) of 2-trifluoromethylbenzenesulfonyl chloride in 600 milliliters (mL) of isopropanol was cooled in an ice bath and treated with a solution of 180 mL of pyridine in 200 mL of isopropanol at a rate to keep the internal temperature <10°C. The mixture was maintained at 0-5°C for 24 hours, then treated with 1 liter (L) of ether. The slurry was filtered and the solvent removed from the filtrate in vacuo. To the filtrate was added 800 mL of ether and the resulting mixture was washed with 500 mL portions of 5% aqueous hydrochloric acid, water, saturated aqueous sodium bicarbonate, water, and then brine. The organic phase was dried through calcium sulfate and the solvent removed in vacuo to yield 306 g isopropyl 2-trifluoromethylbenzenesulfonate as an oil.

A solution of 100g of isopropyl 2-trifluoromethylbenzenesulfonate in 1L of dry tetrahydrofuran was cooled to -78°C under a static nitrogen atmosphere. To this was added 257 mL of a 1.6 molar (M) solution of *n*-butyl lithium in hexane at a rate to keep the internal temperature below -70°C. The yellow solution was stirred 2 hours at this temperature, then treated with 46 mL of iodomethane and warmed to 0°C. After stirring 2 hours at 0°C, the mixture was poured into 1L of saturated aqueous ammonium chloride and 1L of ether. The phases were separated and the organic washed with water and brine and dried through sodium sulfonate. Removal of solvent *in vacuo* yielded 83g of an orange oil which was 90% isopropyl 2-trifluoromethyl-6-methylbenzenesulfonate and 10% unreacted isopropyl 2-trifluoromethylbenzenesulfonate.

### Example 1B: Preparation of isopropyl 2-trifluoromethyl-6-methylbenzenesulfonate

A sample of 0.9 g sodium hydride 60% dispersion in mineral oil was washed with hexane to remove mineral oil, then suspended in 27 mL of tetrahydrofuran and cooled to 0°C. The suspension was treated with 3.1 mL isopropanol, then returned to room temperature and stirred until homogeneous. After re-cooling to 0°C, the mixture was treated with 5 g 2-trifluoromethylbenzenesulfonyl chloride. The mixture was poured into 50 mL of ether and 50 mL of water. The organic phase was dried through sodium sulfate and the solvent removed in vacuo to yield 4.04 g isopropyl 2-trifluoromethylbenzenesulfonate as an oil.

A solution of 4 g of isopropyl 2-trifluoromethylbenzenesulfonate in 40 mL of dry tetrahydrofuran was cooled to -78°C under a static nitrogen atmosphere. To this was added 10.3 mL of a 1.6 M solution of *n*-butyl lithium in hexane at a rate to keep the internal temperature below -70°C. The yellow solution was stirred 2 hours at this temperature, then treated with 1.8 mL of iodomethane and warmed to 0°C. After stirring 2 hours at 0°C, the mixture was poured into 40 mL of saturated aqueous ammonium chloride and 40 mL of ether. The phases were separated and the organic washed with water and brine and dried through sodium sulfate. Removal of solvent in vacuo yielded 3.3 g of an orange oil which was 90% isopropyl 2-trifluoromethyl-6-methylbenzenesulfonate and 10% unreacted isopropyl 2-trifluoromethylbenzenesulfonate.

### Example 1C: Preparation of isopropyl 2-trifluoromethyl-6-methylbenzenesulfonate

A solution of 6.2 g of 2-trifluoromethylbenzenesulfonyl chloride and 0.3 g of 4-dimethylaminopyridine in 30 mL of methylene chloride was cooled to 5°C. A solution of 2.2 g isopropanol and 3.7 g of triethylamine in 20 mL of methylene chloride was prepared and added dropwise to the solution of the sulfonyl chloride. The reaction mixture was returned to room temperature and allowed to stir for 3 hours. The mixture was treated with 200 mL of ether and washed with 60 mL of water. After removal of solvent in vacuo, 5.9 g of isopropyl 2-trifluoromethylbenzenesulfonate was isolated.

A solution of 4 g of isopropyl 2-trifluoromethylbenzenesulfonate in 40 mL of dry tetrahydrofuran was cooled to -78°C under a static nitrogen atmosphere. To this was added 10.3 mL of a 1.6 M solution of *n*-butyl lithium in hexane at a rate to keep the internal temperature below -70°C. The yellow solution was stirred 2 hours at this temperature, then treated with 1.8 mL of iodomethane and warmed to 0°C. After stirring 2 hours at 0°C, the mixture was poured into 40 mL of saturated aqueous ammonium chloride and 40 mL of ether. The phases were separated and the organic washed with water and brine and dried through sodium sulfate. Removal of solvent in vacuo yielded 3.3 g of an orange oil which was 90% isopropyl 2-trifluoromethyl-6-methylbenzenesulfonate and 10% unreacted isopropyl 2-trifluoromethylbenzenesulfonate.

### Example 2: Preparation of ethyl 2-trifluoromethyl-6-methylbenzenesulfonate

Ethyl 2-trifluoromethylbenzenesulfonate was made from 2-trifluoromethylbenzenesulfonyl chloride and treated first with butyl lithium and then with iodomethane in a manner analogous to that set forth above in Example 1A to yield ethyl 2-trifluoromethyl-6-methylbenzenesulfonate.

### Example 3: Preparation of ethyl 2-trifluoromethyl-6-ethylbenzenesulfonate

Ethyl 2-trifluoromethylbenzenesulfonate was made from 2-trifluoromethylbenzenesulfonyl chloride and treated first with butyl lithium and then with iodoethane in a manner analogous to that set forth above in Example 1A to yield ethyl 2-trifluoromethyl-6-ethylbenzenesulfonate.

### Example 4: Preparation of isopropyl 2-chloro-6-ethylbenzenesulfonate

Isopropyl 2-chlorobenzenesulfonate was made from 2-chlorobenzenesulfonyl chloride and treated first with butyl lithium and then with iodoethane in a manner analogous to that set forth above in Example 1A to yield isopropyl 2-chloro-6-ethylbenzenesulfonate.

### Example 5: Preparation of isopropyl 2-trifluoromethoxy-6-methylbenzenesulfonate

Isopropyl 2-trifluoromethoxybenzenesulfonate was made from 2-trifluoromethoxybenzenesulfonyl chloride and then treated first with butyl lithium and then with iodomethane in a manner analogous to that set forth above in Example 1A to yield isopropyl 2-trifluoromethoxy-6-methylbenzenesulfonate.

### Example 6: Preparation of isopropyl 2-trifluoromethyl-6-iodobenzenesulfonate

Isopropyl 2-trifluoromethylbenzene sulfonate was treated first with butyl lithium and then with 2,2,2-trifluoroethyl iodide in a manner analogous to that set forth above in Example 1A to yield isopropyl 2-trifluoromethyl-6-iodobenzenesulfonate.

### Example 7: Preparation of isopropyl 2-trifluoromethyl-6-n-propylbenzenesulfonate

Isopropyl 2-trifluoromethylbenzene sulfonate was treated first with butyl lithium and then with iodopropane in a manner analogous to that set forth above in Example 1A to yield isopropyl 2 -trifluoromethyl-6-*n*-propylbenzenesulfonate.

### Example 8: Preparation of isopropyl 2-fluoro-6-methylbenzenesulfonate

Isopropyl 2-fluorobenzenesulfonate was made from 2-fluorobenzenesulfonyl chloride and treated first with butyl lithium and then with iodomethane in a manner analogous to that set forth above in Example 1A to yield isopropyl 2-fluoro-6-methylbenzenesulfonate (formed as a 2:1 mixture with isopropyl 2-fluoro-3-methylbenzenesulfonate as the minor isomer).

### Example 9: Preparation of isopropyl 2-methyl-3,6-difluoroberzenesulfonate

Isopropyl 2,5-difluorobenzenesulfonate was made from 2,5-difluorobenzensulfonyl chloride and treated first with butyl lithium and then with iodomethane in a manner analogous to that set forth above in Example 1C to yield isopropyl 2-methyl-3,6-difluorobenzenesulfonate.

### Example 10: Preparation of isopropyl 2-ethyl-3,6-difluorobenzenesulfonate

Isopropyl 2,5-difluorobenzenesulfonate was made from 2,5-difluorobenzensulfonyl chloride and treated first with butyl lithium and then with iodoethane in a manner analogous to that set forth above in Example 1C to yield isopropyl 2-ethyl-3,6-difluorobenzenesulfonate.

### Example 11: Preparation of isopropyl 2-fluoro-6-trifluoromethoxybenzenesulfonate

Isopropyl 2-trifluoromethoxybenzenesulfonate was made from 2-trifluoromethoxybenzenesulfonyl chloride and treated first with butyl lithium and then with N-fluoro-O-benzenedisulfonimide in a manner analogous to that set forth above in Example 1B to yield isopropyl 2-fluoro-6-trifluoromethoxybenzenesulfonate.

### Example 12: Preparation of isopropyl 2-methyl-6-trifluoromethoxybenzenesulfonate

Isopropyl 2-trifluoromethoxybenzenesulfonate was made from 2-trifluoromethoxybenzenesulfonyl chloride and treated first with butyl lithium and then with iodomethane in a manner analogous to that set forth above in Example 1B to yield isopropyl 2-methyl-6-trifluoromethoxybenzenesulfonate.

### Example 13: Preparation of isopropyl 2-ethyl-6-trifluoromethoxybenzenesulfonate

Isopropyl 2-trifluoromethoxybenzenesulfonate was made from 2-trifluoromethoxybenzenesulfonyl chloride and treated first with butyl lithium and then with iodoethane in a manner analogous to that set forth above in Example 1B to yield isopropyl 2-ethyl-6-trifluoromethoxybenzenesulfonate.

### Example 14: Preparation of isopropyl 2-methoxymethyl-6-trifluoromethylbenzenesulfonate

Isopropyl 2-trifluoromethylbenzenesulfonate was made from 2-trifluoromethylbenzenesulfonyl chloride and treated first with butyl lithium and then with bromomethyl methyl ether in a manner analogous to that set forth in Example 1A to yield isopropyl 2-methoxymethyl-6-trifluoromethylbenzenesulfonate.

### Example 15: Preparation of isopropyl 2-methylthio-6-trifluoromethylbenzenesulfonate

Isopropyl 2-trifluoromethylbenzenesulfonate was prepared from 2-trifluoromethylbenzenesulfonyl chloride and treated first with butyl lithium and then with dimethyl disulfide in a manner analogous to that set forth in Example 1A to yield isopropyl 2-methylthio-6-trifluoromethylbenzenesulfonate.

### Example 16: Preparation of isopropyl 2-ethylthio-6-methylbenzenesulfonate

Isopropyl 2-ethylthiobenzenesulfonate was prepared from 2-ethylthiobenzenesulfonyl chloride and treated first with butyl lithium and then with iodomethane in a manner analogous to that set forth in Example 1A to yield isopropyl 2-ethylthio-6-methylbenzenesulfonate.

### Example 17: Preparation of 2-ethylthio-6-ethylbenzenesulfonate

Isopropyl-2-ethylthiobenzenesulfonate was prepared from 2-ethylthiobenzenesulfonyl chloride and treated first with butyl lithium and then with iodoethane in a manner analogous to that set forth in Example 1A to yield isopropyl 2-ethylthio-6-ethylbenzenesulfonate.

### Example 18: Preparation of isopropyl 2-ethylthio-6-fluorobenzenesulfonate

Isopropyl 2-ethylthiobenzenesulfonate was prepared from 2-ethylthiobenzenesulfonyl chloride and treated first with butyl lithium and then with N-fluoro-O-benzenedisulfonimide in a manner analogous to that set forth in Example 1A to yield isopropyl 2-ethylthio-6-fluorobenzenesulfonate.

### Example 19: Preparation of isopropyl 2-methylthio-6-methylbenzenesulfonate

Isopropyl 2-methylthiobenzenesulfonate was prepared from 2-methylthiobenzenesulfonyl chloride and treated first with butyl lithium and then with iodomethane in a manner analogous to that set forth in Example 1A to yield isopropyl 2-methylthio-6-methylbenzenesulfonate.

### Example 20: Preparation of isopropyl 2-ethyl-6-methylthiobenzenesulfonate

Isopropyl 2-methylthiobenzenesulfonate was prepared from 2-methylthiobenzenesulfonyl chloride and treated first with butyl lithium and then with iodoethane in a manner analogous to that set forth in Example 1A to yield isopropyl 2-ethyl-6-methylthiobenzenesulfonate.

### Example 21: Preparation of isopropyl 2-fluoro-6-methylthiobenzenesulfonate

Isopropyl 2-methylthiobenzenesulfonate was prepared from 2-methylthiobenzenesulfonyl chloride and treated first with butyl lithium and then with N-fluoro-O-benzenedisulfonimide in a manner analogous to that set forth in Example 1A to yield isopropyl 2-fluoro-6-methylthiobenzenesulfonate.

### Example 22: Preparation of sodium 2-trifluoromethyl-6-methylbenzenesulfonate

A solution of 83g of crude isopropyl 2-trifluoromethyl-6-methylbenzenesulfonate (which contained about 10% of isopropyl 2-trifluoromethylbenzenesulfonate) in 300 mL of tetrahydrofuran was treated with a solution of 24.8g of 50% aqueous sodium hydroxide in 150 mL of water and heated to reflux for 4 hours, then stirred at room temperature overnight. The mixture was assayed by capillary GC (10m DB-1, 100-250°C at 20°C/min) to ascertain that the isopropyl 2-trifluoromethyl-6-methylbenzenesulfonate was consumed. The tetrahydrofuran was removed *in vacuo*, then the remainder was poured into 100 mL of water and 200 mL of hexane. The aqueous phase was washed with hexane, then the water removed *in vacuo* to yield 71g sodium 2-trifluoromethyl-6-methylbenzenesulfonate as a white solid.

### Examples 23-41

The sodium salts of Examples 23-41 were made from the respective compounds of Examples 3-21 in a manner analogous to that set forth above in Example 22:

| **EX#** | **Compound** |
|---|---|
| 23 | Sodium 2-trifluoromethyl-6-ethylbenzenesulfonate |
| 24 | Sodium 2-chloro-6-ethylbenzenesulfonate |
| 25 | Sodium 2-trifluoromethoxy-6-methylbenzenesulfonate |
| 26 | Sodium 2-trifluoromethyl-6-iodobenzenesulfonate |
| 27 | Sodium 2-trifluoromethyl-6-*n*-propylbenzenesulfonate |
| 28 | Sodium 2-fluoro-6-methylbenzenesulfonate |
| 29 | Sodium 2-methyl-3,6-difluorobenzenesulfonate |
| 30 | Sodium 2-ethyl-3,6-difluorobenzenesulfonate |
| 31 | Sodium 2-fluoro-6-trifluoromethoxybenzenesulfonate |
| 32 | Sodium 2-methyl-6-trifluoromethoxybenzenesulfonate |
| 33 | Sodium 2-ethyl-6-trifluoromethoxybenzenesulfonate |
| 34 | Sodium 2-methoxymethyl-6-trifluoromethylbenzenesulfonate |
| 35 | Sodium 2-methylthio-6-trifluoromethylbenzenesulfonate |
| 36 | Sodium 2-ethylthio-6-methylbenzenesulfonate |
| 37 | Sodium 2-ethylthio-6-ethylbenzenesulfonate |
| 38 | Sodium 2-ethylthio-6-fluorobenzenesulfonate |
| 39 | Sodium 2-methylthio-6-methylbenzenesulfonate |
| 40 | Sodium 2-ethyl-6-methylthiobenzenesulfonate |
| 41 | Sodium 2-fluoro-6-methylthiobenzenesulfonate |

### Example 42: Preparation of 2-trifluoromethyl-6-methylbenzenesulfonyl chloride

A mixture of 290g of sodium 2-trifluoromethyl-6-methylbenzenesulfonate and 500 mL of phosphorous oxychloride was heated to reflux for 5 hours. The mixture was then cooled to 0°C and poured into a large flask containing 2000g of ice and 2L of ether. After stirring for 30 minutes, the phases were separated and the organic phase washed with cold water, cold saturated aqueous sodium carbonate, and then brine. The solvent was removed *in vacuo* to yield 2-trifluoromethyl-6-methylbenzenesulfonyl chloride as a brown oil.

### Examples 43-60

The sulfonyl chlorides of Examples 43-60 were prepared from the respective compounds of Examples 23-41 in a manner analogous to that set forth above for Example 42:

| **EX#** | **Compound** |
|---|---|
| 43 | 2-trifluoromethyl-6-ethylbenzenesulfonyl chloride |
| 44 | 2-chloro-6-ethylbenzenesulfonyl chloride |
| 45 | 2-trifluoromethoxy-6-methylbenzenesulfonyl chloride |
| 46 | 2-trifluoromethyl-6-iodobenzenesulfonyl chloride |
| 47 | 2-trifluoromethyl-6-*n*-propylbenzenesulfonyl chloride |
| 48 | 2-fluoro-6-methylbenzenesulfonyl chloride |
| 49 | 2-methyl-3,6-difluorobenzenesulfonyl chloride |
| 50 | 2-ethyl-3,6-difluorobenzenesulfonyl chloride |
| 51 | 2-fluoro-6-trifluoromethoxybenzenesulfonyl chloride |
| 52 | 2-methyl-6-trifluoromethoxybenzenesulfonyl chloride |
| 53 | 2-ethyl-6-trifluoromethoxybenzenesulfonyl chloride |
| 54 | 2-methylthio-6-trifluoromethylbenzenesulfonyl chloride |
| 55 | 2-ethylthio-6-methylbenzenesulfonyl chloride |
| 56 | 2-ethylthio-6-ethylbenzenesulfonyl chloride |
| 57 | 2-ethylthio-6-fluorobenzenesulfonyl chloride |
| 58 | 2-methylthio-6-methylbenzenesulfonyl chloride |
| 59 | 2-ethyl-6-methylthiobenzenesulfonyl chloride |
| 60 | 2-fluoro-6-methylthiobenzenesulfonyl chloride |

The composition and NMR data for the compounds of Examples 1-9, 11-15, 22-26, 28, 32, 34 and 49-52 are set forth below in Table 1. All spectra were recorded at 200 MHz in CDCl₃ with TMS (tetramethylsilane) as an internal standard unless otherwise noted.

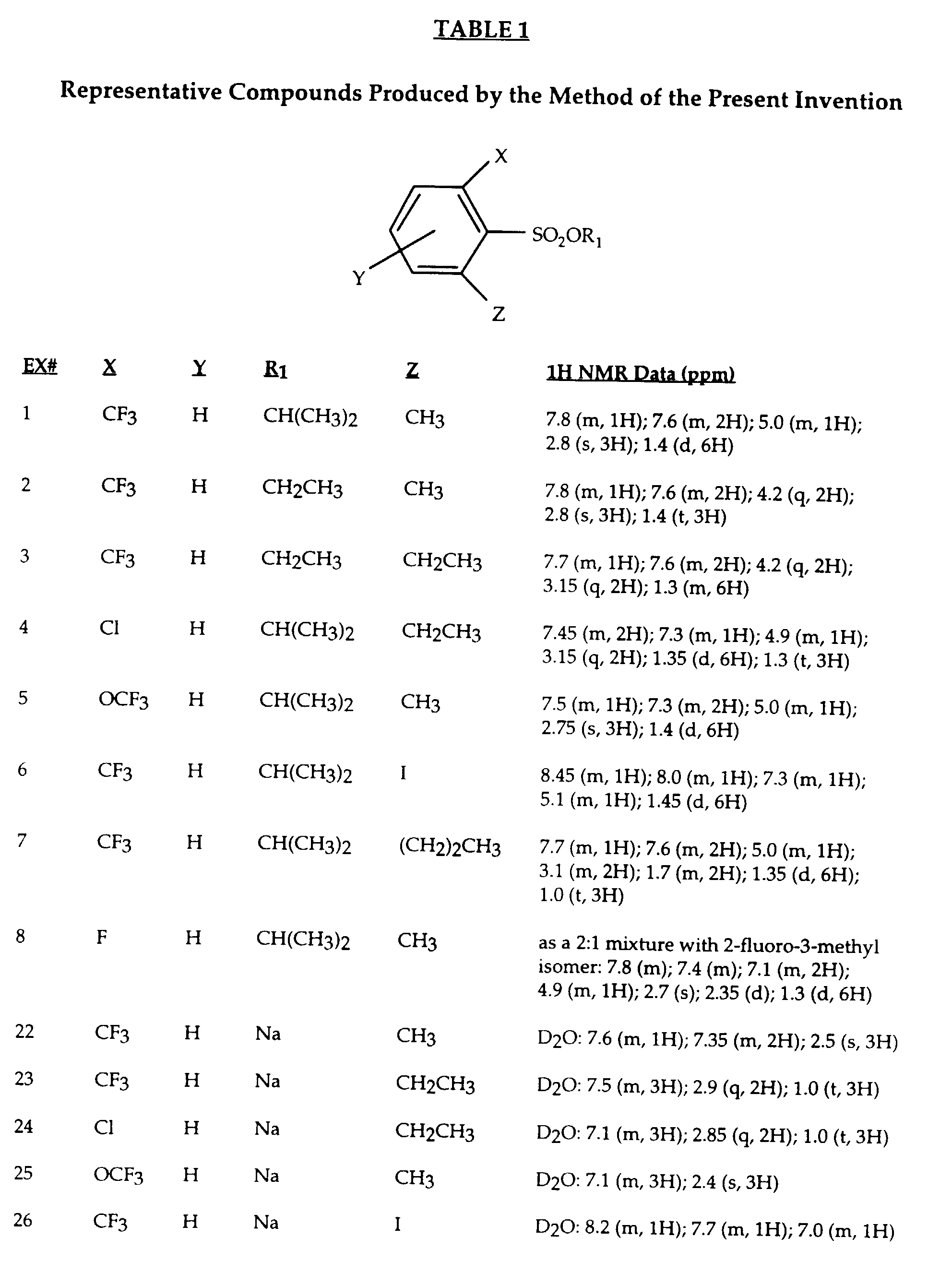

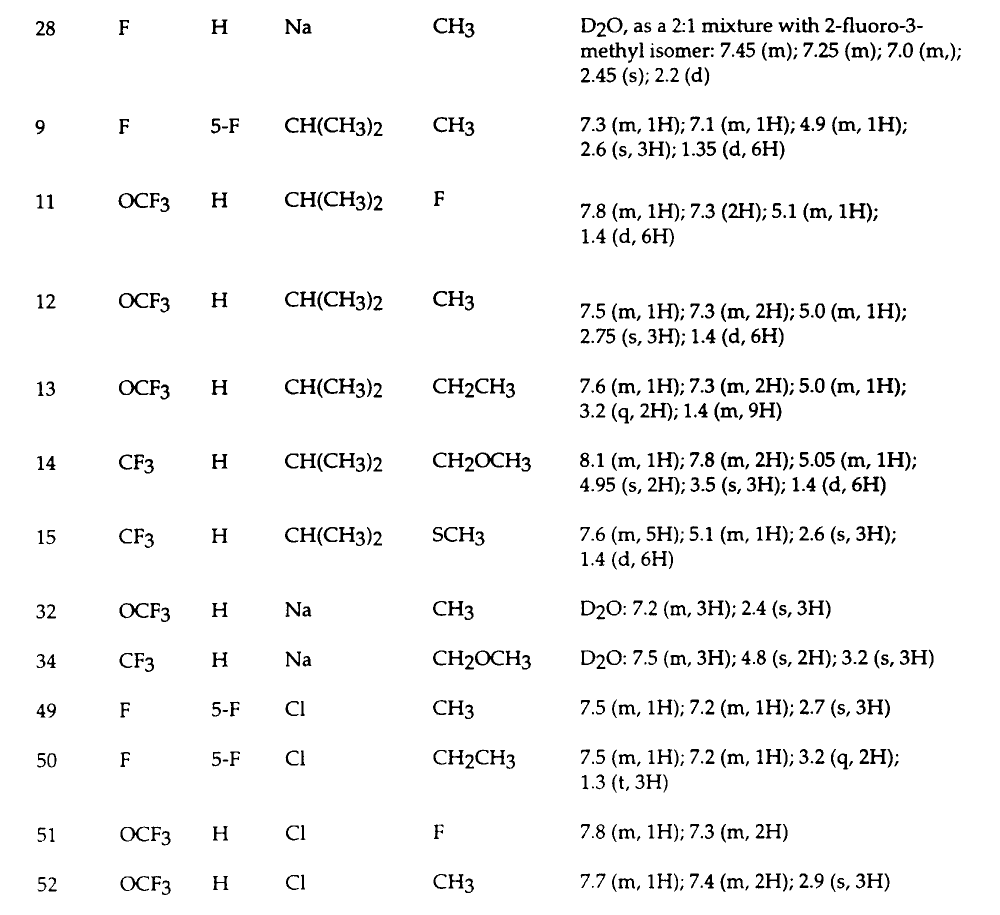

### Example 61: Preparation of di-O-isopropyl P-[[(2-trifluoromethyl-6-methylphenyl)sulfonyloxy]methyl]phosphonate

A mixture of 100 mL of toluene, 11.6 g of 50% aqueous sodium hydroxide solution, and 10 mL of water was cooled in an ice bath and treated with 1.3 g of benzyltriethylammonium chloride and 12.0 g of diisopropyl P-(hydroxymethyl)phosphonate (prepared as in US-A- 5,272,128) and stirred vigorously. To this was added a solution of 15 g of the compound of Example 42 (2-trifluoromethyl-6-methylbenzenesulfonyl chloride) in 10 mL of toluene. The cooling bath was removed and the mixture stirred for one hour, then the layers were separated. The organic phase was washed with water (2 x 100 mL) and brine, then dried through calcium sulfate to yield 23 g of di-O-isopropyl P-[[(2-trifluoromethyl-6-methylphenyl)sulfonyloxy]methyl]phosphonate as a brown oil.

### Example 62: Preparation of O-isopropyl-O-methyl P-[[(2-trifluoromethyl-6-methylphenyl)sulfonyloxy]methyl]phosphonate

A suspension of 11 g of phosphorus oxychloride in 100 mL of methylene chloride was treated with 20.1 g of di-O-isopropyl P-[[(2-trifluoromethyl-6-methylphenyl)sulfonyloxy]methyl]phosphonate in four portions, at a rate to keep the temperature below 28°C. After stirring overnight, the solvent was removed in vacuo to yield 19.5 g of crude O-isopropyl P-[[(2-trifluoromethyl-6-methylphenyl)sulfonyloxy]methyl]phosphonoyl chloride. A solution of 10 g of O-isopropyl P-[[(2-trifluoromethyl-6-methylphenyl)sulfonyloxy]methyl]phosphonoyl chloride in 50 mL of methylene chloride was treated with 0.1 g of dimethylaminopyridine and cooled to -50 °C. A solution of 1.06 mL of dry methanol and 4.2 mL of triethylamine in 10 mL of methylene chloride was added dropwise to the first solution, then the mixture was allowed to return to room temperature and stirred overnight. The mixture was washed with water (2 x 50 mL) and brine and dried through calcium sulfate. Removal of solvent yielded 7.6 g of O-isopropyl-O-methyl P-[[(2-trifluoromethyl-6-methylphenyl)sulfonyloxy]methyl]phosphonate as a yellow oil.

The composition and NMR data for the compounds of Examples 61 and 62 are set forth below in Table 2. The spectra were recorded at 200 MHz in CDCl₃ with TMS as an internal standard.

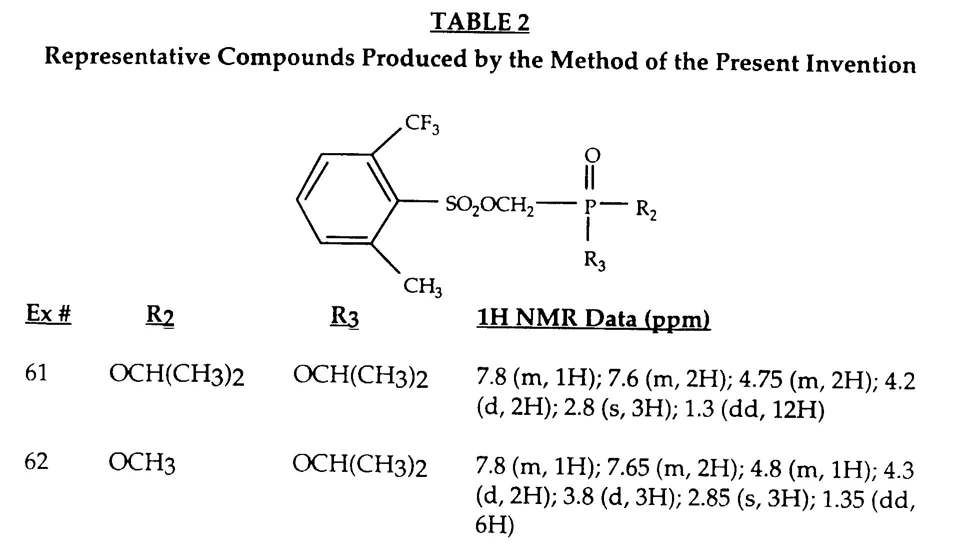

## Claims

1. A method for making a substituted benzene compound which comprises: reacting a 2-substituted benzenesulfonate, or a 2-substituted benzenesulfonate further substituted in the 3, 4 or 5-position, with a lithium compound to form an intermediate compound; and reacting said intermediate compound with an electrophile, selected from the group consisting of alkyl halides, haloalkyl alkyl ethers, aldehydes, ketones, alkyl sulfates, boron esters, alkyl disulfides, phenyl disulfide, deuterium oxide, dimethylformamide, N-formylpiperidine, carbon dioxide, trialkylsilyl chlorides, and sources of positive halogens, to form a 2,6-disubstituted benzenesulfonate or a 2,6-disubstituted benzenesulfonate further substituted in the 3, 4 or 5-position, said 2-substituted benzenesulfonate or said 2-substituted benzenesulfonate further substituted in the 3,4 or 5-position being a compound of the formula 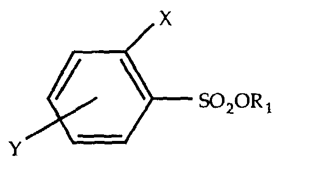 wherein:
R₁ is alkyl, cycloalkyl or phenyl;
X is any group, selected from the group consisting of fluoro, chloro, alkoxy, haloalkyl, haloalkoxy, alkyl, alkylthio, haloalkylthio and N,N-dialkylcarboxamide, that is not reactive with the lithium compound under the reaction conditions used; and
Y is any group, selected from the group consisting of a hydrogen atom, fluoro, chloro, alkoxy, haloalkyl, haloalkoxy, alkylthio and haloalkylthio, in the 3, 4 or 5-position that is not reactive with the lithium compound under the reaction conditions used.

2. A method as claimed in claim 1 wherein: R₁ is (C₂-C₅)alkyl.

3. A method as claimed in claim 2, wherein:
R₁ is isopropyl;
X is fluoro, chloro, (C₁-C₃)alkoxy, halo(C₁-C₃)alkyl, halo(C₁-C₃)alkoxy, (C₁-C₄)alkyl, (C₁-C₄)alkylthio, halo(C₁-C₄)alkylthio, or N,N-di(C₂-C₄)alkylcarboxamide; and
Y is a hydrogen atom, fluoro, chloro, (C₁-C₃)alkoxy, halo(C₁-C₃)alkyl, halo(C₁-C₃)alkoxy, (C₁-C₄)alkylthio, or halo(C₁-C₄)alkylthio.

4. A method as claimed in claim 3, wherein:
X is chloro, fluoro, methoxy, trifluoromethyl, pentafluoroethyl, methylthio, ethylthio or trifluoromethoxy; and
Y is a hydrogen atom, chloro, fluoro, methoxy, trifluoromethyl, trifluoromethoxy, pentafluoroethyl, methylthio, ethylthio, or trifluoromethylthio.

5. A method as claimed in claim 1, wherein a 2-substituted benzenesulfonate is reacted with a lithium compound to form the intermediate compound; and the intermediate compound is reacted with an electrophile to form a 2,6-disubstituted benzenesulfonate.

6. A method as claimed in any preceding claim, wherein the lithium compound comprises elemental lithium, (C₁-C₆)alkyl lithium or an aryl lithium.

7. A method as claimed in claim 6, wherein the lithium compound is selected from the group consisting of elemental lithium, methyl lithium, butyl lithium, hexyl lithium, phenyl lithium and thienyl lithium.

8. A method as claimed in any preceding claim, wherein the electrophile is selected from the group consisting of deuterium oxide, dimethylformamide, N-formylpiperidine, carbon dioxide, iodomethane, iodoethane, iodopropane, bromomethyl methyl ether, formaldehyde, benzaldehyde, benzophenone, dimethylsulfate, trimethyl borate, triisopropyl borate, methyl disulfide, ethyl disulfide, phenyl disulfide, trimethylsilyl chloride, N-fluorobenzenesulfonimide, N-fluoro-O-benzenedisulfonimide, N-fluoropyridinium salts, N-chlorosuccinimide and 2,2,2-trifluoroethyl iodide.

9. A method as claimed in any preceding claim, which further comprises desulfonating the 2,6-disubstituted benzenesulfonate or the 2,6-disubstituted benzenesulfonate further substituted in the 3, 4 or 5-position to form a 1,3-disubstituted benzene or a 1,3-disubstituted benzene further substituted in the 4, 5 or 6-position.

10. A method as claimed in any of claims 1 to 8, which further comprises hydrolyzing the 2,6-disubstituted benzenesulfonate, or the 2,6-disubstituted benzenesulfonate further substituted in the 3,4 or 5-position, to form a 2,6-disubstituted benzenesulfonic acid or a salt of a 2,6-disubstituted benzenesulfonic acid, or a 2,6-disubstituted benzenesulfonic acid further substituted in the 3,4 or 5-position or a salt of a 2,6-disubstituted benzenesulfonic acid further substituted in the 3,4 or 5-position.

11. A method as claimed in any of claims 1 to 8, wherein the method produces a mixture comprising the 2,6-disubstituted benzenesulfonate or the 2,6-disubstituted benzenesulfonate further substituted in the 3,4 or 5-position, and the 2-substituted benzenesulfonate or the 2-substituted benzenesulfonate further substituted in the 3, 4 or 5-position, and the method further comprises: isolating the product mixture from the unreacted material by selectively hydrolyzing the 2,6-disubstituted benzenesulfonate or the 2,6-disubstituted benzenesulfonate further substituted in the 3,4 or 5-position of the product mixture to form a salt of the 2,6-disubstituted benzenesulfonate or a salt of the 2,6-disubstituted benzenesulfonate further substituted in the 3,4 or 5-position; partitioning the product mixture between an aqueous phase and an organic phase; and isolating the 2,6-disubstituted benzenesulfonate or the 2,6-disubstituted benzenesulfonate further substituted in the 3,4 or 5-position salt from the aqueous phase.

12. A method as claimed in claim 10, which further comprises converting the salt of a 2,6-disubstituted benzenesulfonic acid or of a 2,6-disubstituted benzenesulfonic acid further substituted in the 3,4 or 5-position to a 2,6-disubstituted benzenesulfonyl chloride or a 2,6-disubstituted benzenesulfonyl chloride further substituted in the 3,4 or 5-position.

13. A method as claimed in claim 12, which further comprises reacting the 2,6-disubstituted benzenesulfonyl chloride, or the 2,6-disubstituted benzenesulfonyl chloride further substituted in the 3,4 or 5-position, with a hydroxymethylphosphorus compound to form a phosphosulfonate compound.

14. A method as claimed in claim 13, wherein:
the hydroxymethylphosphorus compound has the structural formula 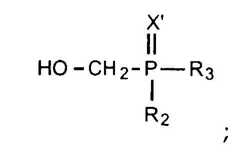 the phosphosulfonate compound has the structural formula 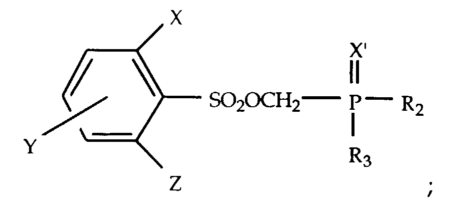
X is fluoro, chloro, alkoxy, haloalkyl, haloalkoxy, alkyl, alkylthio, haloalkylthio, or N,N-dialkylcarboxamide;
Y is a hydrogen atom, fluoro, chloro, alkoxy, haloalkyl, haloalkoxy, alkylthio, or haloalkylthio;
Z is alkyl, methyleneoxy, substituted methyleneoxy, boronic acid, alkylthio, a deuterium atom, formyl, carboxyl, trialkylsilyl or halo;
X' is oxygen or sulfur; and
R₂ and R₃ are each independently alkyl, alkoxy, alkylthio, alkenyloxy, alkynyloxy, haloalkoxy, cyanoalkoxy, alkoxyalkoxy, cycloalkyloxy, cycloalkylalkoxy, alkylideneiminooxy, chloro, amino, phenyl or phenoxy groups, wherein the alkyl, alkoxy, alkylthio, alkenyloxy, alkynyloxy, cycloalkyloxy, cycloalkylalkoxy, alkylideneiminooxy, amino, phenyl and phenoxy groups are unsubstituted or substituted; or when R₂ and R₃ are both alkoxy, R₂ and R₃ may, together with the phosphorus atom, form a six-membered oxygen-containing ring.

## Patentansprüche

1. Verfahren zum Herstellen einer substituierten Benzolverbindung, welches umfaßt: Umsetzen eines 2-substituierten Benzolsulfonats oder eines 2-substituierten Benzolsulfonats, das in der 3-, 4- oder 5-Position weiter substituiert ist, mit einer Lithiumverbindung zur Bildung einer Zwischenverbindung, und Umsetzen der Zwischenverbindung mit einem Elektrophil, ausgewählt aus der Gruppe, bestehend aus Alkylhalogeniden, Haloalkylalkylethern, Aldehyden, Ketonen, Alkylsulfaten, Borestern, Alkyldisulfiden, Phenydisulfid, Deuteriumoxid, Dimethylformamid, N-Formylpiperidin, Kohlendioxid, Trialkylsilylchloriden und Quellen positiver Halogene, zur Bildung eines 2,6-disubstituierten Benzolsulfonats oder eines 2,6-disubstituierten Benzolsulfonats, das in der 3-, 4- oder 5-Position weiter substituiert ist, wobei das 2-substituierte Benzolsulfonat oder das 2-substituierte Benzolsulfonat, das in der 3-, 4- oder 5-Position weiter substituiert ist, eine Verbindung der Formel 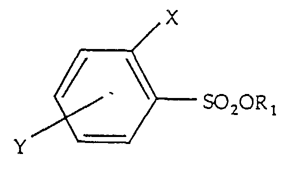 ist, wobei
R₁ Alkyl, Cycloalkyl oder Phenyl bedeutet,
X jede Gruppe, ausgewählt aus der Gruppe, bestehend aus Fluor, Chlor, Alkoxy, Haloalkyl, Haloalkoxy, Alkyl, Alkylthio, Haloalkylthio und N,N-Dialkylcarboxamid, die mit der Lithiumverbindung bei den angewendeten Reaktionsbedingungen nicht reaktiv ist, bedeutet, und
Y jede Gruppe, ausgewählt aus der Gruppe, bestehend aus einem Wasserstoffatom, Fluor, Chlor, Alkoxy, Haloalkyl, Haloalkoxy, Alkylthio und Haloalkylthio in der 3-, 4- oder 5-Position, die mit der Lithiumverbindung bei den angewendeten Reaktionsbedingungen nicht reaktiv ist, bedeutet.

2. Verfahren nach Anspruch 1, wobei R₁ (C₂-C₅)Alkyl bedeutet.

3. Verfahren nach Anspruch 2, wobei
R₁ Isopropyl bedeutet,
X Fluor, Chlor, (C₁-C₃)Alkoxy, Halo(C₁-C₃)alkyl, Halo(C₁-C₃)alkoxy, (C₁-C₄)Alkyl (C₁-C₄)Alkylthio, Halo(C₁-C₄)alkylthio oder N,N-Di(C₂-C₄)alkylcarboxamid bedeutet, und
Y ein Wasserstoffatom, Fluor, Chlor, (C₁-C₃)Alkoxy, Halo(C₁-C₃)alkyl, Halo(C₁-C₃)alkoxy, (C₁-C₄)Alkylthio oder Halo(C₁-C₄)alkylthio bedeutet.

4. Verfahren nach Anspruch 3, wobei
X Chlor, Fluor, Methoxy, Trifluormethyl, Pentafluorethyl, Methylthio, Ethylthio oder Trifluormethoxy bedeutet, und
Y ein Wasserstoffatom, Chlor, Fluor, Methoxy, Trifluormethyl, Trifluormethoxy, Pentafluorethyl, Methylthio, Ethylthio oder Trifluormethylthio bedeutet.

5. Verfahren nach Anspruch 1, wobei ein 2-substituiertes Benzolsulfonat mit einer Lithiumverbindung zur Bildung einer Zwischenverbindung umgesetzt wird, und die Zwischenverbindung mit einem Elektrophil zur Bildung eines 2,6-disubstituierten Benzolsulfonats umgesetzt wird.

6. Verfahren nach einem vorhergehenden Anspruch, wobei die Lithiumverbindung elementares Lithium, (C₁-C₆)Alkyllithium oder ein Aryllithium umfaßt.

7. Verfahren nach Anspruch 6, wobei die Lithiumverbindung aus der Gruppe, bestehend aus elementarem Lithium, Methyllithium, Butyllithium, Hexyllithium, Phenyllithium und Thienyllithium, ausgewählt ist.

8. Verfahren nach einem vorhergehenden Anspruch, wobei das Elektrophil aus der Gruppe, bestehend aus Deuteriumoxid, Dimethylformamid, N-Formylpiperidin, Kohlendioxid, Methyliodid, Ethyliodid, Propyliodid, Brommethylmethylether, Formaldehyd, Benzaldehyd, Benzophenon, Dimethylsulfat, Trimethylborat, Triisopropylborat, Methyldisulfid, Ethyldisulfid, Phenyldisulfid, Trimethylsilylchlorid, N-Fluorbenzolsulfonimid, N-Fluor-o-benzoldisulfonimid, N-Fluorpyidiniumsalzen, N-Chlorsuccinimid und 2,2,2-Trifluorethyliodid, ausgewählt ist.

9. Verfahren nach einem der vorhergehenden Ansprüche, das weiter das Desulfonieren des 2,6-disubstituierten Benzolsulfonats oder des 2,6-disubstituierten Benzolsulfonats, das in der 3-, 4- oder 5-Position weiter substituiert ist, zur Bildung eines 1,3-disubstituierten Benzols oder eines 1,3-disubstituierten Benzols, das in der 4-, 5- oder 6-Position weiter substituiert ist, umfaßt.

10. Verfahren nach einem der Ansprüche 1 bis 8, das weiter das Hydrolysieren des 2,6-disubstituierten Benzolsulfonats oder des 2,6-disubstituierten Benzolsulfonats, das in der 3-, 4- oder 5-Position weiter substituiert ist, zur Bildung einer 2,6-disubstituierten Benzolsulfonsäure oder eines Salzes einer 2,6-disubstituierten Benzolsulfonsäure oder einer 2,6-disubstituierten Benzolsulfonsäure, die in der 3-, 4- oder 5-Position weiter substituiert ist, oder eines Salzes einer 2,6-disubstituierten Benzolsulfonsäure, die in der 3-, 4- oder 5-Position weiter substituiert ist, umfaßt.

11. Verfahren nach einem der Ansprüche 1 bis 8, wobei das Verfahren ein Gemisch, umfassend das 2,6-disubstituierte Benzolsulfonat oder das 2,6-disubstituierte Benzolsulfonat, das in der 3-, 4- oder 5-Position weiter substituiert ist, und das 2-substituierte Benzolsulfonat oder das 2-substituierte Benzolsulfonat, das in der 3-, 4- oder 5-Position weiter substituiert ist, erzeugt, und das Verfahren weiter umfaßt: Isolieren des Produktgemisches vom nicht umgesetzten Material durch selektives Hydrolysieren des 2,6-disubstituierten Benzolsulfonats oder des 2,6-disubstituierten Benzolsulfonats, das in der 3-, 4- oder 5-Position weiter substituiert ist, des Produktgemisches zur Bildung eines Salzes des 2,6-disubstituierten Benzolsulfonats oder eines Salzes des 2,6-disubstituierten Benzolsulfonats, das in der 3-, 4- oder 5-Position weiter substituiert ist, Trennen des Produktgemisches zwischen einer wäßrigen Phase und einer organischen Phase, und Isolieren des 2,6-disubstituierten Benzolsulfonatsalzes oder des Salzes des 2,6-disubstituierten Benzolsulfonats, das in der 3-, 4- oder 5-Position weiter substituiert ist, aus der wäßrigen Phase.

12. Verfahren nach Anspruch 10, das weiter das Umwandeln des Salzes einer 2,6-disubstituierten Benzolsulfonsäure oder einer 2,6-disubstituierten Benzolsulfonsäure, die in der 3-, 4- oder 5-Position weiter substituiert ist, in ein 2,6-disubstituiertes Benzolsulfonylchlorid oder ein 2,6-disubstituiertes Benzolsulfonylchlorid, das in der 3-, 4- oder 5-Position weiter substituiert ist, umfaßt.

13. Verfahren nach Anspruch 12, das weiter das Umsetzen des 2,6-disubstituierten Benzolsulfonylchlorids oder des 2,6-disubstituierten Benzolsulfonylchlorids, das in der 3-, 4- oder 5-Position weiter substituiert ist, mit einer Hydroxymethylphosphorverbindung zur Bildung einer Phosphosulfonatverbindung umfaßt.

14. Verfahren nach Anspruch 13, wobei
die Hydroxymethylphosphorverbindung die Strukturformel 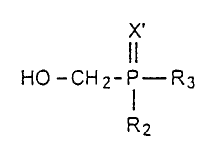 aufweist,
die Phosphosulfonatverbindung die Strukturformel 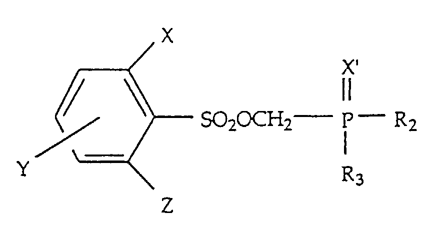 aufweist,
X Fluor, Chlor, Alkoxy, Haloalkyl, Haloalkoxy, Alkyl, Alkylthio, Haloalkylthio oder N,N-Dialkylcarboxamid bedeutet,
Y ein Wasserstoffatom, Fluor, Chlor, Alkoxy, Haloalkyl, Haloalkoxy, Alkylthio oder Haloalkylthio bedeutet,
Z Alkyl, Methylenoxy, substituiertes Methylenoxy, Boronsäure, Alkylthio, ein Deuteriumatom, Formyl, Carboxyl, Trialkylsilyl oder Halogen bedeutet, X' Sauerstoff oder Schwefel bedeutet, und
R₂ und R₃ jeweils unabhängig voneinander Alkyl-, Alkoxy-, Alkylthio-, Alkenyloxy-, Alkinyloxy-, Haloalkoxy-, Cyanalkoxy-, Alkoxyalkoxy-, Cycloalkyloxy-, Cycloalkylalkoxy-, Alkylideniminooxy-, Chlor-, Amino-, Phenyl- oder Phenoxygruppen sind, wobei die Alkyl-, Alkoxy-, Alkylthio-, Alkenyloxy-, Alkinyloxy-, Cycloalkyloxy-, Cycloalkylalkoxy-, Alkylideniminooxy-, Amino-, Phenyl- oder Phenoxygruppen unsubstituiert oder substituiert sind, oder, falls R₂ und R₃ beide Alkoxygruppen sind, R₂ und R₃ zusammen mit dem Posphoratom einen sechsgliedrigen, Sauerstoff enthaltenden Ring bilden können.

## Revendications

1. Procédé de préparation d'un benzène substitué, qui comprend la réaction d'un benzènesulfonate 2-substitué, ou d'un benzènesulfonate 2-substitué qui est par ailleurs substitué sur la position 3, 4 ou 5, avec un composé du lithium pour former un composé intermédiaire ; et la réaction dudit composé intermédiaire avec un électrophile choisi dans le groupe comprenant les halogénures d'alkyle, les oxydes d'halogènalkyle et d'alkyle, les aldéhydes, les cétones, les alkylsulfates, les esters borates, les disulfures d'alkyle, le disulfure de phényle, l'oxyde de deutérium, le diméthylformamide, la N-formylpipéridine, le dioxyde de carbone, les chlorures de trialkylsilyle, et les Sources d'halogènes positifs, pour former un benzènesulfonate 2,6-disubstitué ou un benzènesulfonate 2,6-disubstitué qui est par ailleurs substitué sur la position 3, 4 ou 5, ledit benzène-sulfonate 2-substitué ou ledit benzènesulfonate 2-substitué, par ailleurs substitué sur la position 3, 4 ou 5, étant un composé de formule 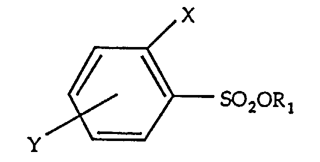 dans laquelle :
R₁ est un radical alkyle, cycloalkyle ou phényle ;
X est un groupe quelconque choisi dans l'ensemble comprenant les groupes fluoro, chloro, alcoxy, halogènalkyle, halogènalcoxy, alkyle, alkylthio, halogènalkylthio et N,N-dialkylcarboxamide, qui n'est pas réactif avec le composé du lithium dans les conditions de réaction utilisées, et
Y est un groupe quelconque choisi dans l'ensemble comprenant l'atome d'hydrogène, les groupes fluoro, chloro, alcoxy, halogènalkyle, halogènalcoxy, alkylthio et halogènalkylthio, sur la position 3, 4 ou 5, qui n'est pas réactif avec le composé du lithium dans les conditions de réaction utilisées.

2. Procédé selon la revendication 1, dans lequel R₁ est un radical alkyle en C₂-C₅.

3. Procédé selon la revendication 2, dans lequel :
R₁ est le groupe isopropyle ;
X est un groupe fluoro, chloro, alcoxy en C₁-C₃, halogènalkyle en C₁-C₃, halogènalcoxy en C₁-C₃, alkyle en C₁-C₄, alkylthio en C₁-C₄, halogènalkylthio en C₁-C₄ ou N,N-di(alkyle en C₂-C₄)carboxamide ; et
Y est un atome d'hydrogène ou un groupe fluoro, chloro, alcoxy en C₁-C₃, halogènalkyle en C₁-C₃, halogènalcoxy en C₁-C₃, alkylthio en C₁-C₄ ou halogènalkylthio en C₁-C₄.

4. Procédé selon la revendication 3, dans lequel :
X est le groupe chloro, fluoro, méthoxy, trifluorométhyle, pentafluorométhyle, pentafluoroéthyle, méthylthio, éthylthio ou trifluorométhoxy ; et
Y est un atome d'hydrogène ou le groupe chloro, fluoro, méthoxy, trifluorométhyle, trifluorométhoxy, pentafluoréthyle, méthylthio, éthylthio ou trifluorométhylthio.

5. Procédé selon la revendication 1, dans lequel on fait réagir un benzènesulfonate 2-substitué avec un composé du lithium pour former le composé intermédiaire, le composé intermédiaire étant mis à réagir avec un électrophile pour former un benzènesulfonate 2,6-disubstitué.

6. Procédé selon l'une quelconque des revendications précédentes, dans lequel le composé du lithium comprend du lithium élémentaire, un alkyllithium en C₁-C₆ ou un aryllithium.

7. Procédé selon la revendication 6, dans lequel le composé du lithium est choisi dans l'ensemble comprenant le lithium élémentaire, le méthyllithium, le butyllithium, l'hexyllithium, le phényllithium et le thiényllithium.

8. Procédé selon l'une quelconque des revendications précédentes, dans lequel l'électrophile est choisi dans l'ensemble comprenant l'oxyde de deutérium, le diméthylformamide, la N-formylpipéridine, le dioxyde de carbone, l'iodométhane, l'iodoéthane, l'iodopropane, l'oxyde de bromométhyle et de méthyle, le formaldéhyde, le benzaldéhyde, la benzophénone, le sulfate de diméthyle, le borate de triméthyle, le borate de triisopropyle, le disulfure de méthyle, le disulfure d'éthyle, le disulfure de phényle, le chlorure de triméthylsilyle, le N-fluorobenzènesulfonimide, le N-fluoro-O-benzènedisulfonimide, les sels de N-fluoropyridinium, le N-chlorosuccinimide et l'iodure de 2,2,2-trifluoroéthyle.

9. Procédé selon l'une quelconque des revendications précédentes, qui comprend en outre la désulfonation du benzènesulfonate 2,6-disubstitué ou du benzènesulfonate 2,6-disubstitué qui est par ailleurs substitué sur la position 3, 4 ou 5, pour former un benzène 1,3-disubstitué ou un benzène 1,3-disubstitué qui est par ailleurs substitué sur la position 4, 5 ou 6.

10. Procédé selon l'une quelconque des revendications 1 à 8, qui comprend en outre l'hydrolyse du benzènesulfonate 2,6-disubstitué, ou du benzènesulfonate 2,6-disubstitué qui est par ailleurs substitué sur la position 3, 4 ou 5, pour former un acide benzènesulfonique 2,6-disubstitué ou un sel d'un acide benzènesulfonique 2,6-disubstitué, ou un acide benzènesulfonique 2,6-disubstitué qui est par ailleurs substitué sur la position 3, 4 ou 5, ou un sel d'un acide benzènesulfonique 2,6-disubstitué qui est par ailleurs substitué sur la position 3, 4 ou 5.

11. Procédé selon l'une quelconque des revendications 1 à 8, dans lequel le procédé produit un mélange comprenant le benzènesulfonate 2,6-disubstitué ou le benzènesulfonate 2,6-disubstitué qui est par ailleurs substitué sur la position 3, 4 ou 5, et le benzènesulfonate 2-substitué ou le benzènesulfonate 2-substitué qui est par ailleurs substitué sur la position 3, 4 ou 5, et le procédé comprend en outre l'isolement du mélange de produits d'avec le matériau n'ayant pas réagi, par hydrolyse sélective du benzènesulfonate 2,6-disubstitué ou du benzène-sulfonate 2,6-disubstitué qui est par ailleurs substitué sur la position 3, 4 ou 5 du mélange de produits, pour former un sel du benzènesulfonate 2,6-disubstitué ou un sel du benzènesulfonate 2,6-disubstitué qui est par ailleurs substitué sur la position 3, 4 ou 5 ; le partage du mélange de produits entre une phase aqueuse et une phase organique ; et l'isolement, d'avec la phase aqueuse, du sel benzènesulfonate 2,6-disubstitué ou benzènesulfonate 2,6-disubstituté qui est par ailleurs substitué sur la position 3, 4 ou 5.

12. Procédé selon la revendication 10, qui comprend en outre la conversion du sel d'un acide benzènesulfonique 2,6-disubstitué ou d'un acide benzènesulfonique 2,6-disubstitué qui est par ailleurs substitué sur la position 3, 4 ou 5, en un chlorure de benzènesulfonyle 2,6-disubstitué ou en un chlorure de benzènesulfonyle 2,6-disubstitué qui est par ailleurs substitué sur la position 3, 4 ou 5.

13. Procédé selon la revendication 12, qui comprend en outre la réaction du chlorure de benzènesulfonyle 2,6-disubstitué ou du chlorure de benzène-sulfonyle 2,6-disubstitué qui est par ailleurs substitué sur la position 3, 4 ou 5, avec un composé de l'hydroxyméthylphosphore, pour former un phosphosulfonate.

14. Procédé selon la revendication 13, dans lequel :
le composé de l'hydroxyméthyolphosphore a la structure développée 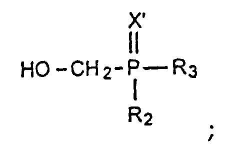 le phosphosulfonate a la structure développée 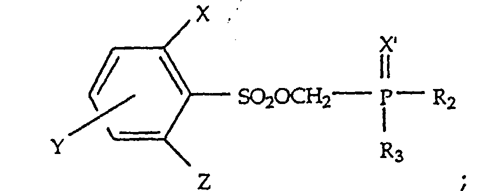
X est un groupe fluoro, chloro, alcoxy, halogènalkyle, halogènalcoxy, alkyle, alkylthio, halogènalkylthio ou N,N-dialkylcarboxamide ;
Y est un atome d'hydrogène ou un groupe fluoro, chloro, alcoxy, halogènalkyle, halogènalcoxy, alkylthio ou halogènalkylthio;
Z est un groupe alkyle, méthylène-oxy, méthylène-oxy substitué, acide boronique, alkylthio, un atome de deutérium, un groupe formyle, carboxyle, trialkylsilyle ou halogéno ;
X' est un atome d'oxygène ou de soufre ; et
R₂ et R₃ représentent chacun indépendamment de l'autre un groupe alkyle, alcoxy, alkylthio, alcényloxy, alcynyloxy, halogènalcoxy, cyanoalcoxy, alcoxyalcoxy, cycloalkyloxy, cycloalkylalcoxy, alkylidèneiminoxy, chlore, amino, phényle ou phénoxy, où les groupes alkyle, alcoxy, alkylthio, alcényloxy, alcynyloxy, cycioalkyloxy, cycloalkylalcoxy, alkylidèneiminoxy, amino, phényle et phénoxy sont non-substitués ou substitués ; ou encore, quand R₂ et R₃ sont tous les deux des groupes alcoxy, R₂ et R₃ peuvent, avec l'atome de phosphore, former un noyau oxygéné à six chaînons.
